# EUROPEAN PATENT APPLICATION

(11) **EP 4 675 339 A1**
(43) Date of publication of application: **07.01.2026**
(21) Application number: 24382726.8
(22) Date of filing: 05.07.2024
(51) Int. Cl.: G02C 3/00, G02C 11/00, A61F 9/02

(54) **LOCATOR ATTACHMENT DEVICE FOR USE WITH EYEGLASSES AND EYEGLASSES WITH TEMPLES WITH LOCATOR ATTACHMENT DEVICE**

(71) Applicant: Pauvazal, S.L., 28223 Pozuelo de Alarcón - Madrid (ES)
(72) Inventor: PAULET VÁZQUEZ, ALBERTO, 28223 MADRID - POZUELO DE ALARCÓN (ES)
(74) Representative: Falcon Morales, Alejandro

(57) **Abstract**

A locator attachment device for use with eyeglasses, wherein the eyeglasses comprise a frame (1) and temples (2) characterized in that it comprises at least one strip of elastic material (3) joining the ends of the temples (2) wherein the strip of elastic material (3) comprises a housing (4) for the locator; A locator attachment device for use with eyeglasses according to claim 1, characterized in that the housing (4) comprises a flange (5) to prevent the locator from slipping out; A device for holding a locator for use with eyeglasses according to claims 1 to 2, characterized in that the housing (4) is circular in shape.

## Description

### Subject matter of the invention

The present invention relates to a locator attachment device for use with a pair of eyeglasses which allows a locating device to be quickly and easily incorporated into a pair of eyeglasses so that the wearer of the eyeglasses is locatable. The present invention falls within the technical field of safety, insofar as the eyeglasses are used as a means of locating a child or an elderly person.

### Technical problem to be solved and background of the invention

Children in crowds can become lost to the adults responsible for them; even if the distance between the adult and the child is small, the visual obstruction and the altered state when a child is lost or an adult cannot locate a child prevent children from being found by their caregivers or parents.

The current state of the art for locating lost children includes GPS tracking bracelets and pendants containing the child's and parent's information.

Older people may also experience moments or circumstances where they get lost, and, if they live alone, their disappearance might not be noticed for hours or days.

In the current state of the art, specifically regarding eyeglasses, strings and cords are used to join the temples of the eyeglasses, allowing them to hang from the user's neck or, in the case of young people, to be adjusted to the child's head, thereby preventing the eyeglasses from falling off and ensuring they remain in position on the child's face.

### Description of the invention

The present invention pertains to a locator attachment device for use with eyeglasses, wherein the eyeglasses comprise a frame and temples such that the device pertaining to the invention comprises at least one strip of elastic material joining the ends of the temples wherein the strip of elastic material comprises a housing for the locator.

In one embodiment of a locator attachment device for use with eyeglasses, as pertaining to the invention, the housing comprises a flange to prevent the locator from slipping out.

In one embodiment of the invention, the housing is circular, specifically designed for locators of the same shape.

The present invention also pertains to eyeglasses with a locator attachment device as previously defined.

### Description of the drawings

To complete the description, a set of drawings in which the following has been represented is attached to this descriptive report, as an integral element of the same:

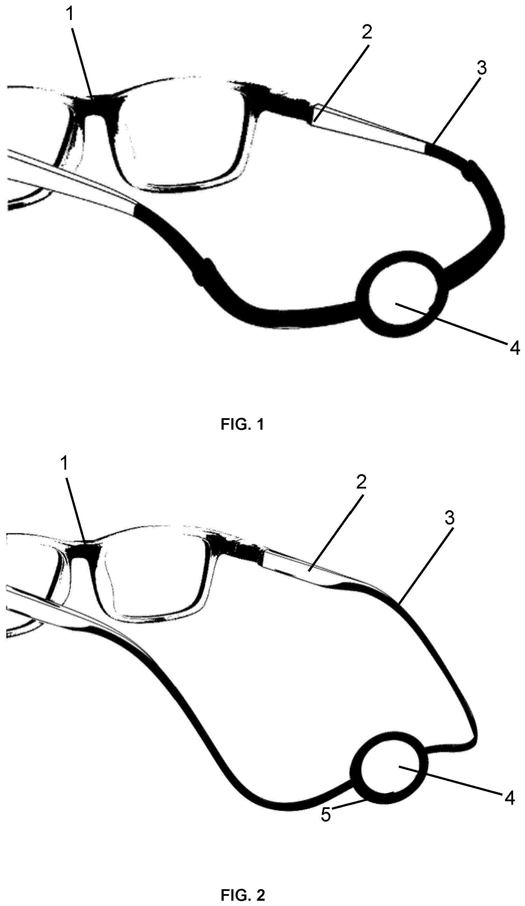

Figure 1. Shows a perspective view of a first embodiment of a device for attaching a locator to eyeglasses.
Figure 2. Shows a perspective view of a second embodiment of a device for attaching a locator to eyeglasses.

### Preferred embodiment of the invention

The invention pertains to a locator attachment device for eyeglasses, enabling the determination of the eyeglasses' location and, consequently, the location of the wearer.

The eyeglasses for use with the device pertaining the invention comprise a frame (1) and temples (2). The device pertaining to the invention comprises at least one strip of elastic material (3) joining the ends of the temples (2) surrounding the head of a user, such that the strip of elastic material (3) comprises a housing for the locator (4). Thus, with the device pertaining to the invention, the locator is incorporated into the eyeglasses in a simple and convenient manner.

The device for attaching a locator for use with eyeglasses pertaining to the invention may, in a second embodiment, be incorporated directly into eyeglasses, being a single piece together with the temples (2) of the eyeglasses, so that the eyeglasses incorporate the locator together with the temples (2) in a unitary piece.

Said housing has a flange (5) to prevent the locator from slipping out of the housing (4).

In one embodiment of the invention, the housing (4) is circular in shape so that it is designed especially for locators having such a shape.

The present invention also pertains to eyeglasses with a locator attachment device as previously defined.

## Claims

1. A locator attachment device for use with eyeglasses, wherein the eyeglasses comprise a frame (1) and temples (2) **characterized in that** it comprises at least one strip of elastic material (3) joining the ends of the temples (2) wherein the strip of elastic material (3) comprises a housing (4) for the locator.

2. A locator attachment device for use with eyeglasses according to claim 1, **characterized in that** the housing (4) comprises a flange (5) to prevent the locator from slipping out.

3. A device for holding a locator for use with eyeglasses according to claims 1 to 2, **characterized in that** the housing (4) is circular in shape.

4. Eyeglasses with a locator attachment device as defined in any one of the preceding claims.
